# EUROPÄISCHE PATENTSCHRIFT

(11) **EP 2 699 213 B1**
(45) Veröffentlichungstag und Bekanntmachung des Hinweises auf die Patenterteilung: **10.05.2017**
(21) Anmeldenummer: 12711409.8
(22) Anmeldetag: 30.03.2012
(51) Int. Cl.: A61F 13/70, A61F 13/505

(54) **FIXIER- UND TRAGVORRICHTUNG ZUM SICHEREN HALTEN EINER WEGWERFBAREN ABSORBIERENDEN INKONTINENZVORLAGE**
FASTENING AND CARRYING DEVICE FOR THE SAFE HOLDING OF A DISPOSABLE ABSORBENT INCONTINENCE PAD
DISPOSITIF DE FIXATION ET DE SUPPORT POUR LE MAINTIEN SECURISE D'UNE GARNITURE ABSORBANTE ET JETABLE POUR INCONTINENCE

(30) Priorität: 20.04.2011 DE 102011007821
(43) Veröffentlichungstag der Anmeldung: 26.02.2014
(73) Patentinhaber: Paul Hartmann AG, 89522 Heidenheim (DE)
(72) Erfinder: PAZ, Rui Miguel, 47800 Krefeld (DE); KESSELMEIER, Ruediger, 89542 Herbrechtingen (DE); MALOWANIEC, D. Krzysztof, 89522 Heidenheim (DE); DRUMEVA-EBERIUS, Albena, 89522 Heidenheim (DE); NAGY, Uwe, 89522 Heidenheim (DE); SWEREV, Maximilian, 86169 Augsburg (DE)
(74) Vertreter: DREISS Patentanwälte PartG mbB
(86) Internationale Anmeldenummer: PCT/EP2012/055877
(87) Internationale Veröffentlichungsnummer: WO 2012/143230

(56) Entgegenhaltungen:
- WO-A1-2004/069122
- WO-A1-2011/044995
- US-A1- 2006 247 599

## Beschreibung

Die Erfindung betrifft eine Fixier- und Tragevorrichtung für eine wegwerfbare absorbierende Inkontinenzvorlage, mit einem mittels Gürtelverschlusselementen auf sich selbst lösbar schließbaren und so eine in Hüftumfangsrichtung durchgehende Hüftöffnung bildenden Hüftgürtel, an den die Inkontinenzvorlage lösbar fixierbar ist, so dass sie im Schrittbereich des Benutzers getragen und nach Gebrauch wieder vom Hüftgürtel entfernt und verworfen werden kann, wobei der Hüftgürtel einen vorderen Bauchbereich, einen hinteren Rückenbereich und einen linken und einen rechten Seitenbereich umfasst und der Hüftgürtel ausgehend von dem Rückenbereich und dem Bauchbereich je einen vorzugsweise symmetrisch zu einer Längsmittelachse vorgesehenen und sich in einer Längsrichtung in Richtung auf den Schrittbereich des Benutzers erstreckenden Latzabschnitt aufweist, der an seiner körperzugewandten Seite vorzugsweise mechanisch und/oder klebend wirkende Verschlusselemente aufweist, die mit komplementären vorzugsweise mechanisch und/oder klebend wirkenden Verschlusselementen auf der körperabgewandten Seite der Inkontinenzvorlage lösbar haftend zusammenwirken.

Eine derartige Fixier- und Tragevorrichtung ist beispielsweise bekannt aus WO 2004/069122 A1. Bei dieser bekannten Fixier- und Tragevorrichtung wird der Hüftgürtel im Bauchbereich auf sich selbst geschlossen. In Seitenbereichen links und rechts zwischen Bauchbereich und Rückenbereich erstreckt sich der Hüftgürtel nach unten und bildet dort jeweils einen latzartigen Ansatz, an den eine verhältnismäßig breit ausladende absorbierende Windeleinheit festgelegt werden kann, wobei auf der körperzugewandten Seite der absorbierenden Windeleinheit beidseits vorn und hinten Verschlusselemente hierfür vorgesehen sind.

Aus EP 0 700 278 B1 ist eine sogenannte Gürtelwindel mit einem auf sich selbst schließbaren verhältnismäßig schmalen Hüftgürtel bekannt, an dessen körperabgewandter Seite eine nach Art einer Windel anmutende absorbierende Einheit festlegbar ist.

Eine gattungsgemäße Fixier- und Tragevorrichtung ergibt sich aus der DE 10 2009 049 463 A1.

US 2006/247599 A1 offenbart eine Fixier- und Tragevorrichtung für eine wegwerfbare absorbierende Inkontinenzvorlage mit einem mittels Gürtelverschlusselementen auf sich selbst lösbar schließbaren und so eine in Hüftumfangsrichtung durchgehende Hüftöffnung bildenden Hüftgürtel an dem die Inkontinenzvorlage lösbar fixierbar ist. Der vorliegenden Erfindung liegt die Aufgabe zugrunde, eine Fixier- und Tragevorrichtung der eingangs genannten Art zu schaffen, die ein sicheres Anlegen und Positionieren der Fixier- und Tragevorrichtung sowie einen Wechsel der Inkontinenzvorlage erlaubt.

Diese Aufgabe wird ausgehend von einer Fixier- und Tragevorrichtung der genannten Art erfindungsgemäß dadurch gelöst, dass die aufeinander fixierten Gürtelverschlusselemente bei angelegter Fixier- und Tragevorrichtung eine größere oder gleiche Haltekraft aufweisen, als die am Latzabschnitt angeordneten Verschlusselemente auf den komplementären Verschlusselementen der Inkontinenzvorlage.

Hierdurch ist der Vorteil begründet, dass auch bei einem Wechsel der Inkontinenzvorlage der Hüftgürtel nicht ungewollt durch die auf den Hüftgürtel beim Lösen der Verschlusselemente wirkenden Kräfte mit gelöst wird. Die Haltekräfte der Gürtelverschlusselemente sind daher vorzugsweise zumindest geringfügig größer als die Haltekräfte der Verschlusselemente auf den komplementären Verschlusselementen der Inkontinenzvorlage. Dabei ist jedoch darauf zu achten, dass die Haltekräfte der Gürtelverschlusselemente so ausgebildet sind, dass ein Öffnen und Schließen derselben möglich ist, sofern dies vorgesehen sein soll. Alternativ kann auch nur ein einmaliges Schließen der Gürtelverschlusselemente vorgesehen sein. In diesem Fall muss der Hüftgürtel dann zum Entfernen vom Benutzer zerstört werden, wozu zum Beispiel entsprechende Reißlinien vorgesehen sein können.

Unter Haltekraft sollen hierbei die Scher- und/oder die Peelkräfte verstanden werden.

Dabei kann insbesondere vorgesehen sein, dass die Peelkräfte der Gürtelverschlusselemente dem 2- bis 15-fachen, vorzugsweise dem 3- bis 10-fachen der Peelkräfte der Verschlusselemente auf der Inkontinenzvorlage entsprechen.

Weiterhin kann vorgesehen sein, dass die Scherkräfte der Gürtelverschlusselemente dem 2,5- bis 25-fachen, vorzugsweise dem 3-bis 15-fachen, weiter bevorzugt dem 5- bis 12-fachen der Scherkräfte der Verschlusselemente auf der Inkontinenzvorlage entsprechen.

Durch den in Richtung auf den Schrittbereich des Benutzers erstreckten Latzabschnitt bildet der Hüftgürtel die grundsätzliche Konfiguration eines Höschens, welches zur Vollendung der Höschenform nur noch durch einen Schrittbereich ergänzt zu werden braucht. Dieser Schrittbereich wird bei der erfindungsgemäßen Fixier- und Tragevorrichtung durch die absorbierende Inkontinenzvorlage gebildet, wobei der betreffende hintere und vordere Latzabschnitt mit der körperabgewandten Seite der Inkontinenzvorlage lösbar haftend zusammenwirkt, und zwar unter Verwendung von vorzugsweise mechanisch und/oder klebend wirkenden Verschlusselementen.

Besonders bevorzugt weist die Inkontinenzvorlage zwei Längs- und zwei Querkanten auf, wobei im an der Fixier- und Tragevorrichtung festgelegten Zustand die Querkanten der Inkontinenzvorlage vorzugsweise im Wesentlichen einer Querkante, insbesondere der dem Schrittbereich abgewandten, also proximalen Querkante des Hüftgürtels entsprechen. Das heißt, die proximale Kante des Bauch- oder Rückenbereichs fällt vorzugsweise mit einer Querkante der Inkontinenzvorlage in angelegten Zustand zusammen. Im Falle einer konturierten proximalen Querkante des Hüftgürtels, beispielsweise durch einen Bauchausschnitt, läuft die proximale Kante parallel zur Hüftumfangsrichtung durch den distalsten Punkt dieser Konturierung. Auf diese Weise kann einfach eine Markierung bereitgestellt werden, anhand derer die optimale Anlegung des Hüftgürtels sowie der Inkontinenzvorlage am Hüftgürtel erreicht werden kann und somit auch für die jeweilige Größe optimale Positionierung der Verschlusselemente, um einen auf der einen Seite optimalen Aufspanneffekt der Materialabschnitte und damit der dem Träger zugewandten Lage zu erreichen und auf der anderen Seite einen Kontakt der Verschlusselemente mit dem Benutzer zu verhindern. Vorzugsweise sind die Verschlusselemente in dieser Position 100 mm bis 180 mm, vorzugsweise 120 mm bis 170 mm, weiter bevorzugt 140 mm bis 160 mm im Bereich des vorderen Latzabschnitts und vorzugsweise 170 mm bis 250 mm, vorzugsweise 190 mm bis 240 mm, weiter bevorzugt 210 mm bis 230 mm im Bereich des hinteren Latzabschnitts von der proximalen Querkante des Hüftgürtels entfernt.

Weiterhin umfasst die Inkontinenzvorlage eine Rückenschicht (Backsheet oder Außenseite), die vorzugsweise flüssigkeitsundurchlässig ist und eine körperzugewandte Schicht (Topsheet), die vorzugsweise flüssigkeitsdurchlässig ist sowie einen zwischen diesen beiden Schichten eingeschlossenen absorbierenden Kern, der eine äußeren Rand als Begrenzung aufweist.

Die Breite der Inkontinenzvorlage an der breitesten Stelle des Produktes beträgt von 200 mm bis 400 mm, vorzugsweise 220 mm bis 320 mm, die Breite des absorbierenden Kerns beträgt von 180 mm bis 380 mm, vorzugsweise 200 mm bis 300 mm. So beträgt beispielsweise die Breite der Inkontinenzvorlage MoliForm® Premium Soft Light (Größe 1) 260 mm, die Breite des absorbierenden Kerns 242 mm, bei MoliForm® Premium Soft Extra (Größe 3) beträgt die Breite der Vorlage 300 mm, die Breite des absorbierenden Kerns 260 mm.

Der Hüftgürtel umfasst in Hüftumfangsrichtung einen Bauchbereich sowie einen Rückenbereich und zwei Seitenbereiche, die jeweils eine Erstreckung in Hüftumfangsrichtung aufweisen und sich in Hüftumfangsrichtung aneinander anschließen bei Bildung eines geschlossenen Hüftgürtels.

In Längsrichtung besitzen Bauch- und Rückenbereich sowie die Seitenbereiche eine proximale und eine distale Querkante, die gerade oder gekrümmt, insbesondere auch nur bereichsweise gekrümmt ausgebildet sein können, zur Ausbildung einer Konturierung.

Schließlich wird die distale Erstreckung des Bauch- und Rückenbereichs und damit die distale Querkante des Bauch- und Rückenbereichs definiert durch eine gedachte Linie in Querrichtung am proximalsten Punkt der distalen Begrenzung des Hüftgürtels, beispielsweise eine Tangente. Der proximalste Punkt kann dabei in Hüftumfangsrichtung im Bauch-, Rücken- oder Seitenbereich liegen. Dabei ist die Fläche proximal dieser distalen Querkante ein Teil des Bauch- bzw. Rückenbereichs und die Fläche distal dieser distalen Querkante ein Teil des jeweiligen Latzabschnitts.

Als Längsrichtung des Hüftgürtels wird dabei die Längsrichtung an einem stehenden Benutzer definiert, die Querrichtung ist dabei senkrecht hierzu und fällt mit der Hüftumfangsrichtung zusammen. Die Längsrichtung der Inkontinenzvorlage entspricht der Richtung deren Längserstreckung, wobei die Querrichtung hierzu senkrecht verläuft.

Dabei erweist es sich als vorteilhaft, wenn die Verschlusselemente des Latzabschnitts vor der Ingebrauchnahme auf der körperzugewandten Seite des Latzabschnitts bzw. auf den Bauch- oder Rückenbereich eingeschlagen und in dieser Konfiguration leicht lösbar an der körperzugewandten Seite des Latzabschnitts oder des Bauch- oder Rückenbereichs haftend sind. Es kann dann bevorzugt vorgesehen sein, dass die Haltekräfte der Gürtelverschlusselemente aufeinander größer oder gleich groß wie die Haltekräfte der Verschlusselemente des Latzabschnitts auf den Latzabschnitten bzw. dem Bauch- oder Rückenbereich sind. Hierdurch wird der Vorteil erreicht, dass bei Lösen der Verschlusselemente von dem Latzabschnitt oder vom sonstigen Hüftgürtel und der damit einhergehenden Kraft es nicht zu einem ungewollten Öffnen der Gürtelverschlusselemente kommt.

Zusätzlich kann diese Faltung der Latzabschnitte durch Fixierpunkte festgelegt werden. Dies kann zum Beispiel mittels einer Ultraschallverbindung, insbesondere kleiner separater Schweißpunkte erfolgen, die im Randbereich der überlappenden Lagen befestigt werden, aber auch mittels Klebeverbindungen, insbesondere Klebepunkten. Andere Arten der Anhaftung sind ebenfalls umfasst. Die Öffnungskraft dieser zusätzlichen Fixierung der Latzfaltung liegt signifikant unterhalb der Materialfestigkeit des Hüftgürtels und zerstört beim Auffalten der Latzabschnitte nicht das Material des Hüftgürtels. In diesem Fall müssen die Haltekräfte der Fixierpunkte kleiner oder gleich groß wie die Haltekräfte der Gürtelverschlusselemente aufeinander sein.

Besonders bevorzug ist dabei, wenn die Gürtelverschlusselemente eine Peelkraft aufweisen, die dem 2- bis 25-fachen, vorzugsweise dem 5- bis 15-fachen, der Peelkräfte der Verschlusselemente auf dem Latzabschnitt oder dem Bauch- oder Rückenbereich entspricht. Dabei sind beim Lösen der eingeschlagenen und anhaftenden Verschlusselemente vom Latzabschnitt oder dem übrigen Hüftgürtel lediglich Peelkräfte relevant, da beim Ablösen keine Scherkräfte zum Trage kommen.

Durch die Abstimmung der Haltekräfte aufeinander wird ein Produktversagen verhindert, das z.B. in einem ungewollten Lösen der Inkontinenzvorlage bzw. vermeidbarem zusätzlichem Aufwand beim Trennen der Inkontinenzvorlage vom Hüftgürtel und einem dadurch bedingten Öffnen des Hüftgürtels oder zu einem Öffnen des Hüftgürtels beim Lösen der Verschlusselemente vom Latzabschnitt oder vom Bauch- oder Rückenbereich führt.

Darüber hinaus muss jedoch die Inkontinenzvorlage sicher am Hüftgürtel gehalten werden, auch wenn es zu einem Positionieren des Latzabschnitts zum Benutzer kommt. D. h. die Haltekraft der Verschlusselemente muss ausreichend groß sein, um ein solches ungewolltes Lösen der Inkontinenzvorlage zu verhindern.

Besonders bevorzugt ist es, wenn die Haltekraft der Verschlusselemente der Latzabschnitte auf den komplementären Verschlusselementen der Inkontinenzvorlage größer als die maximale definierte Gewichtskraft einer Inkontinenzvorlage im beladenen Zustand ist. Hierbei wird insbesondere ein maximaler Beladungszustand definiert. Dieser kann zum Beispiel dadurch angegeben werden, dass für eine spezifische Inkontinenzvorlage eine produktbezogene, praxisrelevante Saugleistung, beispielsweise in Form der "Absorption before leakage" gemäß EDANA Prüfmethode WSP 354.0 ermittelt wird, die dann als maximale Gewichtskraft für die spezifische Inkontinenzvorlage zugrundegelegt wird und wobei die Haltekräfte der Verschlusselemente größer diesem Wert sind. Der "Absorption before leakage"-Wert für Inkontinenzvorlagen beträgt zwischen 350 und 1000 ml und liegt je nach Saugstärke der Inkontinenzvorlage vorzugsweise bei 350 ml oder 450 ml oder 500 ml oder 650 ml oder 750 ml oder 850 ml oder 1000 ml. Dies entspricht einer Gewichtskraft 3,4 N bis 9,8 N, vorzugsweise von 3,4N, 4,4N, 4,9N, 6,4N, 7,4N, 8,3N oder 9,8N, die durch die Verschlusselemente zu halten ist. Hinzu kommt das Eigengewicht der Vorlage und weiterhin Kräfte, die in der Tragesituation durch den Benutzer auf die Verschlusselemente wirken. Daher sollten die Verschlusselemente eine Haltekraft im Bereich zwischen 2 und 15 N, vorzugsweise zwischen 2,5 und 12 N, weiter bevorzugt zwischen 3 und 11 N besitzen. Da in der Fixier- und Tragevorrichtung zwei Verschlusselemente auf den Latzabschnitten die Gewichtskraft zu gleichen Teilen aufnehmen, muss jedes Verschlusselement zwischen 1 und 7,5 N, vorzugsweise zwischen 1,25 und 6 N, weiter bevorzugt zwischen 1,5 und 5,5 N tragen.

Als theoretische Obergrenze für eine mögliche Gewichtskraft kann die so genannte ISO-Saugleistung (ISO 11948-1), bei der der gesamte Saugkörper vollständig gesättigt und ausgelastet ist, angenommen werden. Diese liegt vorzugsweise zwischen 1000 ml und 3500 ml, bevorzugt zwischen 1200 ml und 3000 ml, so dass eine maximale Gewichtskraft von ca. 10 bis 36 N bzw. bevorzugt von ca. 12 bis 32 N inklusive des Eigengewichtes der Vorlage durch die Verschlusselemente aufgenommen werden muss bzw. für das einzelne Verschlusselement 5 bis 18 N vorzugsweise 6 bis 16 N.

Hierdurch wird erreicht, dass das Gewicht der Inkontinenzvorlage, das insbesondere bei mobilen Benutzern eine Kraft nach unten ausübt, nicht zu einem ungewollten Lösen der Inkontinenzvorlage oder der Gürtelverschlusselemente führt.

Zur Verbesserung der Passform und für eine einfache Handhabung erweist es sich als vorteilhaft, wenn der betreffende Latzabschnitt sich in Richtung auf den Schrittbereich verjüngt.

Weiter erweist es sich als vorteilhaft, wenn die Verschlusselemente des Latzabschnitts an einem schrittzugewandten Ende des Latzabschnitts vorgesehen sind. Darüber hinaus können am hinteren und/oder vorderen Latzabschnitt ein oder mehrere weitere Verschlusselemente vorgesehen sein, die zwischen dem schrittzugewandten Ende und dem Hüftgürtel positioniert sind und die der zusätzlichen Fixierung der Inkontinenzvorlage dienen.

Weiter erweist es sich als vorteilhaft, wenn die vorzugsweise mechanisch und/oder klebend wirkenden Verschlusselemente des Latzabschnitts, insbesondere am schrittzugewandten Ende des Latzabschnitts, auf einem streifenförmigen und quer zur Längsrichtung erstreckten Materialabschnitt vorgesehen sind, der an den Latzabschnitt angefügt ist. Auf diese Weise kann der Latzabschnitt über seine gesamte flächenhafte Erstreckung in Querrichtung in einer bestimmungsgemäßen zum Benutzer symmetrischen Weise an der Außenseite (Backsheet) der Inkontinenzvorlage lösbar festgelegt werden. Der Latzabschnitt kann so weitgehend faltenfrei und wie erwähnt zu den Beinen und zum Schrittbereich bzw. zu einer auf den Benutzer abstellenden Längsmittelachse korrekt ausgebreitet und fixiert werden.

Vorzugsweise haben die mechanisch und/oder klebend wirkenden Verschlusselemente des Latzabschnitts eine Erstreckung in Querrichtung von 8 cm bis 14 cm, vorzugsweise 10 cm bis 12 cm, vorzugsweise 11 cm und vorzugsweise eine Erstreckung in Längsrichtung von 0,5 cm bis 5,0 cm, vorzugsweise 1,0 cm bis 4,0 cm, vorzugsweise von 1,0cm bis 3,0cm und vorzugsweise 1,5 cm. Damit ist eine ausreichend große Fläche für die Haftverbindung der mechanisch und/oder klebend wirkenden Verschlusselemente des Latzabschnitts mit der Außenseite der Inkontinenzvorlage für einen sicheren Produktsitz gewährleistet.

Die Außenseite der Inkontinenzvorlage ist dabei so auszubilden, dass sie mit den Verschlusselementen des Latzabschnittes korrespondiert, das heißt, bei einem mechanischen Verschlusselement in Form von Haken wird die Außenseite der Inkontinenzvorlage vorzugsweise durch eine schlaufenbildende Komponente, vorzugsweise durch ein Vlies, insbesondere die Vlieslage eines Vlies-Folien-Laminates, gebildet, während bei einem klebenden Verschlusselement die Außenseite der Inkontinenzvorlage vorzugsweise durch eine Folienlage gebildet wird, auf der die klebenden Verschlusselemente anhaften können.

Dabei sind grundsätzlich auch Kombinationen aus mechanischen und adhäsiven Verschlusselementen möglich.

Nach einem weiteren Erfindungsgedanken erweist es sich -wie bereits dargelegt - als vorteilhaft, wenn die Verschlusselemente des Latzabschnitts, insbesondere am schrittzugewandten Ende, vor der Ingebrauchnahme auf die körperzugewandte Seite des Latzabschnitts eingeschlagen und in dieser Konfiguration leicht lösbar an der körperzugewandten Seite des Latzabschnitts haftend sind. Auf diese Weise sind die an dem Latzabschnitt vorgesehenen, insbesondere mechanisch und/ oder klebend wirkenden Verschlusselemente während des Anlegens des Hüftgürtels, insbesondere bei pflegebedürftigen Personen, so lange deaktiviert, wie sie nicht zum Festlegen der Inkontinenzvorlage benötigt werden. Sie behindern dabei nicht das Anlegen des Hüftgürtels, indem sie am Hüftgürtel oder an anderen Komponenten wie Bettlaken, Patientenunterlagen oder dergleichen, festhaken. In der Praxis kann es sich nämlich als vorteilhaft erweisen, wenn der vordere und/oder hintere Latzabschnitt auf sich selbst und/oder auf den Hüftgürtel gefaltet ist und die Faltung erst nach Anlegen des Hüftgürtels und der Inkontinenzvorlage geöffnet wird, um die Vorlage zu fixieren.

Im Hinblick auf die praktische Handhabbarkeit erweist es sich auch als vorteilhaft, wenn der Hüftgürtel nur in einem Bereich öffenbar und schließbar und die Gürtelverschlusselemente in diesem Bereich an freien Endabschnitten des Hüftgürtels vorgesehen sind. Der Hüftgürtel ist also langgestreckt und hat zwei Endabschnitte, die mittels der Gürtelverschlusselemente aufeinander schließbar sind, um den Hüftgürtel um den Körperumfang des Benutzers herum anlegen zu können.

In Weiterbildung dieses Erfindungsgedankens erweist es sich als vorteilhaft, wenn im anderen Seitenbereich, in dem der Gürtel also nicht öffenbar und schließbar ist, Sekundärverschlusselemente vorgesehen sind, mittels derer eine Umfangslänge des Hüftgürtels einstellbar und so zumindest der vordere Latzabschnitt symmetrisch zum Schritt des Benutzers positionierbar ist. Vorzugsweise sind die Sekundärverschlusselemente so ausgebildet, dass sie im angelegten Zustand der Fixier- und Tragevorrichtung in der Ansicht optisch und/oder haptisch nicht von den Gürtelverschlusselementen unterscheidbar sind. Es kann sich jedoch auch als vorteilhaft erweisen, wenn die Sekundärverschlusselemente von den Gürtelverschlusselementen optisch und/oder haptisch unterscheidbar sind, um auf die unterschiedliche Funktion der Verschlusselemente hinzuweisen. Dies kann beispielsweise durch unterschiedliche Farben, Formen, Textur oder auf andere Weise erfolgen. Die Gürtelverschlusselemente und die Sekundärverschlusselemente sind dabei vorteilhafter Weise beidseits des Bauchabschnitts und/oder in Seitenbereichen des Hüftgürtels vorgesehen. - Beim Anlegen des Hüftgürtels führt die Pflegeperson den geöffneten langgestreckten Hüftgürtel bei einem liegenden Patienten unter dessen Körper auf Hüft- oder Rückenhöhe hindurch. Sodann wird der Gürtel zur Bildung der geschlossenen Hüftöffnung mittels der Gürtelverschlusselemente auf sich selbst geschlossen. In diesem Zustand ist der Gürtel zwar angelegt, jedoch noch nicht durch Aktivierung der Sekundärverschlusselemente in seine optimale Passform gebracht. Beispielsweise wird in diesem Zustand der bauchseitige Latzabschnitt durch den Zug in Hüftumfangsrichtung, der durch das Schließen der Gürtelverschlusselemente ausgeübt wird, zu der betreffenden Seite hin gezogen. Dem kann nun durch Einstellen der optimalen Hüftumfangslänge und einer geeigneten Spannung in Hüftumfangsrichtung begegnet werden, indem die Sekundärverschlusselemente entsprechend optimal eingestellt werden. Mittels dieser Sekundärverschlusselemente werden also - wie bereits ausgeführt - nicht zwei offene freie Enden aufeinander geschlossen, sondern es wird die Umfangslänge des Hüftgürtels gegebenenfalls verstellt.

Die Sekundärverschlusselemente können in verschiedener Weise verwirklicht sein. Nach einer bevorzugten Ausführungsform der Erfindung wird vorgeschlagen, dass eine Komponente der Sekundärverschlusselemente auf einem an den Hüftgürtel angefügten Materialabschnitt vorgesehen ist und die andere Komponente an einer Außenseite des Hüftgürtels selbst vorgesehen ist. Solchenfalls wird also der Hüftgürtel als solcher nicht verändert, sondern er erhält ein zusätzliches Element in Form des die Sekundärverschlusselemente tragenden Materialabschnitts, der in an sich beliebiger Weise auf das Material des Hüftgürtels aufgebracht werden kann, beispielsweise durch Aufnähen, Aufkleben, Aufsiegeln, Ultraschallschweißen oder dergleichen übliche Fügeverfahren.

Es erweist sich auch als vorteilhaft, wenn sich die Gürtelverschlusselemente und/oder die Sekundärverschlusselemente über im Wesentlichen die gesamte Erstreckung des Hüftgürtels in Längsrichtung, also über die gesamte Breite des Hüftgürtels, erstrecken. Auf diese Weise kann beim Einstellen der Sekundärverschlusselemente ein gleichmäßiger Zug über die gesamte Breite des Gürtels auf den Gürtel ausgeübt werden.

Es ist auch denkbar, dass sich die Gürtelverschlusselemente und/oder die Sekundärverschlusselemente zu ihrem freien Ende hin verjüngen oder verschmälern, um ein Reiben der Ecken dieser Verschlusselemente auf der Haut des Benutzers zu minimieren.

In einer alternativen Ausführungsform ist es darüber hinaus denkbar, dass die Gürtelverschlusselemente und/oder die Sekundärverschlusselemente sich nicht über im Wesentlichen die gesamte Breite des Hüftgürtels erstrecken. In einem solchen Fall ist es besonders vorteilhaft, wenn die Quererstreckung der Gürtelverschlusselemente und/oder der Sekundärverschlusselemente größer ist als ihre Längserstreckung.

Vorzugsweise stehen die Gürtelverschlusselemente und/oder die Sekundärverschlusselemente über den Seitenrand des Hüftgürtels um mindestens 30 mm, insbesondere um mindestens 40 mm und weiter insbesondere um mindestens 50 mm in Querrichtung über. Die Erstreckung der Gürtelverschlusselemente und/oder die Sekundärverschlusselemente in Längsrichtung der Fixier- und Tragevorrichtung beträgt insbesondere mindestens 25 mm, weiter insbesondere mindestens 35 mm und weiter insbesondere mindestens 45 mm. Damit ergibt sich ein bevorzugtes Verhältnis von Quererstreckung zu Längserstreckung von 30 zu 25, vorzugsweise von 40 zu 35 und insbesondere von 50 zu 45. Insbesondere kann vorgesehen sein, dass die Gürtelverschlusselemente und/oder die Sekundärverschlusselemente trapezförmig ausgebildet sind, insbesondere mit einem Seitenverhältnis von 50mm in Querrichtung zu 35/30mm in Längsrichtung, wobei die kürzere Seite des Trapezes die freie Seite der Verschlusselemente bildet. Damit ist eine ausreichend große Fläche für die Haftverbindung der Gürtelverschlusselemente und/oder der Sekundärverschlusselemente mit der Außenseite des Hüftgürtels für einen sicheren Produktsitz gewährleistet.

Dabei können die Gürtelverschlusselemente und/oder die Sekundärverschlusselemente sogenannte Anfass- oder Griffabschnitte umfassen oder mit diesen verbunden sein. Die Anfass- oder Griffabschnitte sind vorzugsweise am freien Ende der Gürtel- oder Sekundärverschlusselemente vorgesehen und weisen keine Verschlussmittel z.B. in Form von mechanischen und/oder klebenden Elementen auf. Hierdurch wird die Anfassbarkeit der Verschlussmittel verbessert.

In Weiterbildung der Erfindung wird vorgeschlagen, die Gürtelverschlusselemente und/oder die Sekundärverschlusselemente zumindest abschnittsweise elastisch auszubilden.

Zum Verkürzen der Umfangslänge des Gürtels unter Verwendung der Sekundärverschlusselemente wird dabei vorzugsweise eine Z-förmige Faltung des Gürtels durch Schließen der Sekundärverschlusselemente bewirkt, die durch den dabei entstehenden Druck des Hüftgürtels gegen die Körperoberfläche des Benutzers und/oder durch Reibung der aneinander anliegenden Abschnitte der Z-förmigen Faltung gehalten wird.

Im Hinblick auf die Schaffung einer guten Passform und eines hohen Tragkomforts erweist es sich als vorteilhaft, wenn der Hüftgürtel mindestens einen elastischen Abschnitt, vorzugsweise in jedem Seitenbereich einen elastischen Abschnitt umfasst, so dass der Hüftgürtel in Hüftumfangsrichtung elastisch dehnbar ist.

Es erweist sich weiter als vorteilhaft, wenn der Hüftgürtel im Bauchbereich und/oder im Rückenbereich im Wesentlichen undehnbar ausgebildet ist.

In einer alternativen Ausführungsform kann es sich jedoch auch als vorteilhaft erweisen, wenn sich ein oder mehrere elastische Abschnitte im Bauchbereich und/oder Rückenbereich befinden, vorzugsweise in Form einer keilförmigen, trapezförmigen oder viereckigen Elastifizierung.

Eine besonders bevorzugte alternative Ausführungsform weist zwei trapezförmige Elastifizierungen im Rückenbereich auf, die insbesondere als elastisch dehnbare Materialabschnitte ausgebildet sind, die jeweils in den Bereichen des Rückenbereichs vorgesehen sind, die an die Seitenbereiche angrenzen und zwischen sich einen nicht elastischen Bereich einschließen. Dabei können die beiden parallelen Kanten der elastischen Materialabschnitte in Längsrichtung angeordnet sein und die beiden weiteren Kanten mit einer proximalen Querkante des Hüftgürtels und mit einer auf den Schrittbereich gerichteten Kante des Rückenbereichs zusammenfallen. Je nach Breite der elastischen Bereich kann vorgesehen sein, dass diese sich in Längsrichtung bis in den Latzabschnitt, insbesondere den hinteren Latzabschnitt erstrecken.

Im Hinblick auf die Bemessung des Hüftgürtels erweist es sich als vorteilhaft, wenn der Hüftgürtel an vorzugsweise jeder Stelle eine Erstreckung in Längsrichtung von wenigstens 5 cm, insbesondere von wenigstens 6 cm, insbesondere von wenigstens 7 cm, insbesondere von wenigstens 8 cm aufweist. Hierbei ist die Abmessung des Hüftgürtels in Längsrichtung, also die Gürtelbreite quer zu seiner Umfangserstreckung, und zwar außerhalb des vorderen oder hinteren Latzabschnitts gemeint. Die Erstreckung des Hüftgürtels in Längsrichtung ist vorzugsweise höchstens 25 cm. Besonders bevorzugt ist die Erstreckung im Bauchbereich geringer als im Rückenbereich, und der obere Rand des Hüftgürtels ist vorzugsweise leicht konturiert, um einen Bauchausschnitt zu bilden, der die Passform des Produkts verbessert sowie ein Rollen oder Umschlagen des Gürtels im Bauchbereich verhindert.

Hinsichtlich der Bemessung des Latzabschnitts erweist es sich als vorteilhaft, wenn der Latzabschnitt eine Erstreckung in Längsrichtung über eine schrittzugewandte distale Querkante des Bauch- oder Rückenbereichs in distaler Richtung auf den Schrittbereich von wenigstens 5 cm, insbesondere von wenigstens 6 cm, insbesondere von wenigstens 7 cm, insbesondere von wenigstens 8 cm, insbesondere von wenigstens 9 cm, insbesondere von wenigstens 10 cm, insbesondere von höchstens 20 cm, insbesondere von 10 - 18 cm, insbesondere von 10 - 16 cm aufweist, wobei der vordere und hintere Latzabschnitt die gleiche oder eine unterschiedliche Erstreckung haben können. Die Erstreckung des Hüftgürtels in Längsrichtung beträgt im Bereich des Latzabschnitts vorne bei konturiertem Bauchausschnitt vorzugsweise 13 cm bis 19 cm, vorzugsweise 15 cm bis 17 cm, insbesondere 16 cm, im Bereich des Latzabschnitts hinten vorzugsweise 20 cm bis 27 cm, vorzugsweise 22 cm bis 25 cm, insbesondere 23,5 cm. Bei konturiertem Bauchausschnitt wurde dieser Wert ausgehend vom distalsten (tiefsten) Punkt der proximalen Querkante des Bauchbereichs bestimmt.

Die Erstreckung des Hüftgürtels in Querrichtung, das heisst, in Hüftumfangsrichtung beträgt zwischen 75 und 175 cm , vorzugsweise für die Größe S zwischen 50 und 90 cm, für die Größe M zwischen 80 und 120 cm, für die Größe L zwischen 100 und 140 cm, für die Größe XL zwischen 120 und 160 cm sowie für die Größe XXL zwischen 140 und 190 cm und orientiert sich an den Kleiderkonfektionsgrößen.

Nach einer bevorzugten Ausführungsform der Erfindung ist der betreffende Latzabschnitt einstückig mit dem Bauchbereich und/oder mit dem Rückenbereich des Hüftgürtels ausgebildet. Separat angestückte Latzabschnitte aus dem gleichen Material wie der Hüftgürtel oder aus einem anderen Material als der Hüftgürtel sind aber ebenfalls denkbar.

Der Bauchbereich und/oder Rückenbereich des Hüftgürtels ist vorzugsweise von einem Vliesmaterial oder Vliesverbundmaterial, insbesondere von einem im Wesentlichen undehnbaren Vliesmaterial oder Vliesverbundmaterial, gebildet.

Weiter erweist es sich als besonders vorteilhaft, wenn ein elastisch dehnbarer Seitenbereich des Hüftgürtels mit einem im Wesentlichen undehnbaren Bauchbereich und einem im Wesentlichen undehnbaren Rückenbereich unlösbar verbunden ist. Vorzugsweise umfasst jeder Seitenbereich einen elastisch dehnbaren Abschnitt. In einer alternativen Ausführungsform kann es sich als vorteilhaft erweisen, wenn Teile des Rückenbereichs, insbesondere die seitlichen Teile des Rückenbereichs, die an die Seitenbereiche angefügt sind, elastische ausgebildet sind.

Darüber hinaus erweist es sich als vorteilhaft, wenn der vordere und/oder hintere Latzabschnitt farbig ausgebildet ist, wobei die Abschnitte vorzugsweise unterschiedlich farbig sind, um als visuelle Anlegehilfe zu dienen.

Weiterhin ist es vorteilhaft, wenn auf dem vorderen und/oder hinteren Latzabschnitt eine Markierung angebracht ist, die als Positionierhilfe für die Inkontinenzvorlage dient, derart, dass bei korrekt angelegter Inkontinenzvorlage und Fixier- und Tragevorrichtung die Markierung auf dem vorderen und/oder hinteren Latzabschnitt mit einer auf der äußeren Lage der Inkontinenzvorlage sichtbaren Markierung in Einklang gebracht ist. Beispielsweise kann eine Ausnehmung mittig am schrittzugewandten Rand des vorderen und/oder hinteren Latzabschnitts in Einklang gebracht werden mit einer Markierung z.B. in Form eines zentral in Längsrichtung angeordneten Nässeindikators auf der äußeren Lage der Inkontinenzvorlage. Alternativ kann die Breite des unteren Rand des vorderen und/oder hinteren Latzabschnitts herstellerseitig so gewählt werden, dass diese mit dem Abstand von Markierungen auf der äußeren Lage der Inkontinenzvorlage, wie beispielsweise einer Größen- oder Saugstärken-Markierung, in Einklang gebracht werden kann.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der erfindungsgemäßen Fixier- und Tragevorrichtung für eine wegwerfbare absorbierende Inkontinenzvorlage.

In der Zeichnung zeigt:
Figuren 1 und 2 eine Ausführungsform der erfindungsgemäßen Fixier- und Tragevorrichtung im angelegten Zustand zusammen mit einer wegwerfbaren absorbierenden Inkontinenzvorlage,
Figur 3 eine schematische Draufsicht auf die Fixier- und Tragevorrichtung im geöffneten und auf eine ebene Unterlage ausgebreiteten Zustand und
Figuren 4 bis 6 die Methode zur Ermittlung der Scherkraft.

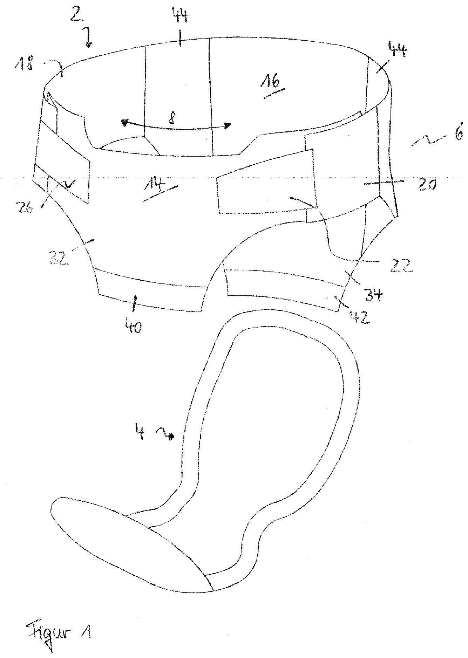

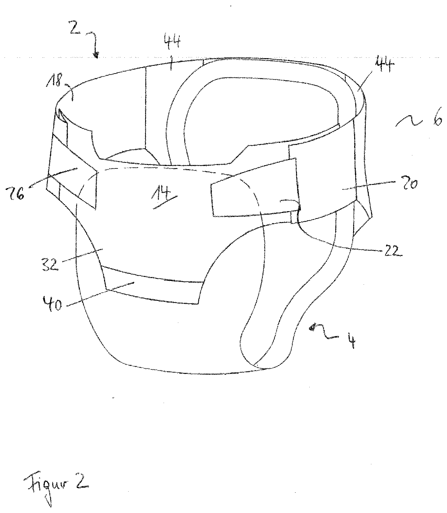

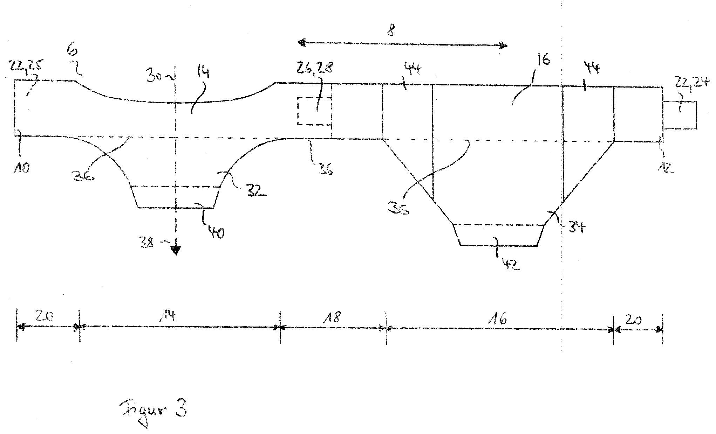

Die Figuren 1 und 2 zeigen perspektivische Ansichten einer insgesamt mit dem Bezugszeichen 2 bezeichneten Fixier- und Tragevorrichtung für eine mit Bezugszeichen 4 angedeutete absorbierende Inkontinenzvorlage, und zwar im an einen Benutzer angelegten Zustand. Die Fixier- und Tragevorrichtung 2 umfasst einen auf sich selbst lösbar schließbaren Hüftgürtel 6, der sich entlang einer Hüftumfangsrichtung 8 erstreckt und zwei freie Endabschnitte 10, 12 (s. Figur 3) aufweist, die zur Bildung der geschlossenen Hüftumfangsform auf sich selbst schließbar sind.

Die Fixier- und Tragevorrichtung 2 bzw. ihr Hüftgürtel 6 umfassen einen vorderen Bauchbereich 14, einen hinteren Rückenbereich 16 sowie einen linken Seitenbereich 18 und einen rechten Seitenbereich 20.

Zum Schließen des Hüftgürtels 6 sind an dessen freien Endabschnitten 10, 12 Gürtelverschlusselemente 22 vorgesehen, die im bevorzugt und beispielhaft dargestellten Fall von mechanisch wirkenden Verschlusselementen in Form eines Haken/Schlaufenverschlusssystems gebildet sind. Dabei ist in den Figuren mit Bezugszeichen 24 die hakenförmige Verschlusskomponente dargestellt, die mit einer schlaufenbildenden Komponente 25 in Form der äußeren Oberfläche des Bauchbereichs 14 lösbar haftend zusammenwirkt. Beispielsweise weist die Außenseite des Bauchbereichs 14 ein Vliesmaterial auf, welches die schlaufenförmige Komponente 25 des Verschlusssystems bildet.

Des Weiteren weist der nur an seinen Endabschnitten 10, 12, also nur an einer Stelle offenbare Hüftgürtel 6 Sekundärverschlusselemente 26 auf, mittels derer die Umfangslänge des Hüftgürtels 6 einstellbar ist. Die Sekundärverschlusselemente 26 sind seitlich am Bauchbereich 14 bzw. am linken Seitenbereich 18 so vorgesehen, dass sie im angelegten Zustand der Fixier- und Tragevorrichtung (s. Figur 1) denselben optischen Eindruck wie die Gürtelverschlusselemente 22 hinsichtlich Ausbildung und Anordnung vermitteln, obschon ihre Funktion verschieden ist. Mittels der Sekundärverschlusselemente 26 werden nämlich keine öffenbaren und voneinander trennbaren Endabschnitte des Hüftgürtels 6 miteinander verbunden, sondern die Sekundärverschlusselemente 26 dienen lediglich dazu, die Umfangslänge des Hüftgürtels 6 an die Tragesituation anzupassen. Hierfür umfassen die Sekundärverschlusselemente 26 einen auf die Außenseite des Hüftgürtels 6 aufgebrachten Materialabschnitt 28, auf dem oder an dem sie selbst vorgesehen sind. Dieser Materialabschnitt 28 erstreckt sich vorzugsweise über die gesamte Längserstreckung des Gürtels (entlang einer Längsmittelachse 30), also die gesamte Gürtelbreite, um gleichmäßig Zugkräfte in den Hüftgürtel einleiten zu können. Bei den Sekundärverschlusselementen 26 handelt es sich wiederum in bevorzugter Weise um mechanisch wirkende Verschlusselemente, insbesondere um Haken/Schlaufenmaterialien. Im beispielhaft dargestellten und bevorzugten Fall sind die Sekundärverschlusselemente 26 derart ausgebildet, dass an dem Materialabschnitt 28 eine hakenbildende Komponente des Verschlusssystems vorgesehen ist, die mit einer schlaufenbildenden Außenseite des Bauchbereichs 14 zusammenwirkt, so wie dies vorausgehend bei den Gürtelverschlusselementen 22 beschrieben wurde.

Als weitere wesentliche Komponente umfasst der Hüftgürtel 6 ausgehend vom Bauchbereich 14 und vom Rückenbereich 16 je einen symmetrisch zu einer Längsmittelachse 30 ausgebildeten vorderen und hinteren Latzabschnitt 32, 34. Die Latzabschnitte 32, 34 sind im beispielhaft bevorzugt dargestellten Fall einstückig mit dem Hüftgürtel 6 ausgebildet; sie erstrecken sich von einer schrittzugewandten oder unteren, distalen Querkante 36 des Hüftgürtels entlang der Längsmittelachse 30 in Richtung 38 auf den Schrittbereich. Sie können seitlich bogenförmig konturiert ausgebildet sein. An ihrem unteren, schrittzugewandten Endbereich umfassen die Latzabschnitte 32, 34 mechanisch wirkende Verschlusselemente 40, 42, und zwar auf der körperzugewandten Seite. Diese mechanisch wirkenden Verschlusselemente 40, 42 wirken lösbar haftend mit komplementären mechanisch wirkenden Verschlusselementen auf der körperabgewandten Seite der Inkontinenzvorlage 4 zusammen, um die Inkontinenzvorlage 4 lösbar, jedoch verliersicher an der Fixier- und Tragevorrichtung 2 festzulegen. Bevorzugtermaßen handelt es sich bei den Verschlusselementen 40, 42 um die hakenbildende Komponente eines mechanisch wirkenden Verschlusssystems, die mit der schlaufenbildenden Komponenten auf der körperabgewandten Seite der Inkontinenzvorlage 4 zusammenwirkt. Die schlaufenbildende Komponente kann dabei in vorteilhafter Weise von einer Vliesbeschichtung, vorzugsweise der Vlieslage eines Vlies-Folien-Laminates, der Inkontinenzvorlage 4 gebildet sein.

Der Hüftgürtel 6 ist vorzugsweise aus Vlies- oder Vliesverbundmaterialien hergestellt. Im beispielhaft dargestellten jedoch bevorzugten Fall ist der Bauchbereich 14 und der Rückenbereich 16 aus einem im Wesentlichen undehnbaren Vliesmaterial oder Vliesverbundmaterial gebildet, dessen Außenseite, die als schlaufenbildende Komponente der Gürtelverschlusselemente 22 bzw. der Sekundärverschlusselemente 26 dient. Die beiden Seitenbereiche 18, 20 sind hingegen elastisch dehnbar ausgebildet, indem sie einen elastisch dehnbaren Materialabschnitt 44 aufweisen, der bestimmungsgemäß unlösbar mit dem undehnbaren Material des Bauchbereichs 14 und Rückenbereichs 16 verbunden ist.

Das Anlegen der Fixier- und Tragevorrichtung 2 zusammen mit der absorbierenden Inkontinenzvorlage 4 geschieht folgendermaßen: Zunächst wird der Hüftgürtel 6 bei bettlägerigen pflegebedürftigen Patienten durch eine Pflegeperson unter dem Körper des Patienten auf Hüfthöhe hindurchgeführt. Der Hüftgürtel 6 wird dabei so positioniert, dass der Rückenbereich 16 unterhalb des Gesäßes und der Bauchbereich 14 ungefähr vorne mittig zu liegen kommen. Sodann werden die freien Endabschnitte 10, 12 des Hüftgürtels 6 übereinander positioniert und mittels der Gürtelverschlusselemente 22 aufeinander geschlossen. Durch anschließendes Positionieren und Schließen des Materialabschnitts 28 und der Sekundärverschlusselemente 26 wird die Länge und Spannung des Hüftgürtels 6 in Umfangsrichtung 8 optimiert, so dass ein gleichmäßiger Zug auf den Bauchbereich 14 links und rechts ausgeübt wird und der Bauchbereich 14 wie auch der Rückenbereich 16 vorzugsweise symmetrisch in Bezug auf den Patienten und möglichst faltenfrei zu liegen kommen. Hierbei ist auch auf ein angenehmes Traggefühl abzustellen. Sodann wird der vordere und hintere Latzabschnitt 32, 34 mit der zuvor oder erst jetzt im Schrittbereich des Patienten positionierten Inkontinenzvorlage 4 unter Verwendung der mechanisch wirkenden Verschlusselemente 40, 42 lösbar haftend verbunden. Auch hierbei ist darauf zu achten, dass die vorderen und hinteren Latzabschnitte 32, 34 unter Ausübung einer gleichmäßigen und moderaten Zug- bzw. Haltekraft auf die Inkontinenzvorlage 4 fixiert werden, damit die Inkontinenzvorlage 4 in ihrer bestimmungsgemäßen symmetrischen Anordnung im Schrittbereich des Patienten verbleibt.

Hinsichtlich der Reihenfolge der vorausgehend geschilderten Schritte zum Anlegen des Hygieneartikels bzw. Inkontinenzsystems ist die Pflegeperson verhältnismäßig frei. Beispielsweise kann es sich in bestimmten Pflegesituationen als vorteilhaft erweisen, dass zunächst die Inkontinenzvorlage im Schrittbereich des Patienten vorpositioniert wird und erst dann der Hüftgürtel 6 auf sich selbst geschlossen wird. Es kann sich auch als zweckmäßig erweisen, dass die Sekundärverschlusselemente 26 erst nach dem Verbinden der Latzabschnitte 32, 34 mit der Inkontinenzvorlage 4 genutzt werden, um die optimale Spannung in Hüftumfangsrichtung 8 vorzugeben.

In den nachfolgend beschriebenen Tests werden die folgenden Materialien verwendet, die insbesondere geeignet sind, als Materialien der Fixier- und Tragvorrichtung und der Inkontinenzvorlage.

### Verwendete Materialien

### Schlaufenbildende Komponente

Die Materialien 1 und 2 (M1 und M2) der Fixier- und Tragevorrichtung stellen alternative Materialien für die Fixier- und Tragevorrichtung dar.

Material 1 (M1) der Fixier- und Tragevorrichtung: 60 gsm thermobonded Karden-/Spinnvlies Laminat (Artikel Nr: 0410600801400), RKW SE Gronau; Düppelstraße 16; 48599 Gronau, Germany
Material 2 (M2) der Fixier- und Tragevorrichtung: 50 gsm Wasserstrahl verfestigtes Spinnvlies (HyJet SB 540), RKW SE Gronau; Düppelstraße 16; 48599 Gronau, Germany
Backsheet der Vorlage: 22 gsm (14 gsm Spinnvlies + 8 gsm PE Folie) Textile Backsheet, Hyfol PE Soft Textile, RKW SE Wasserburg, Alkorstraße 6, 83512 Wasserburg

### Hakenbildende Komponente:

Gürtelverschlusselement 22 und Sekundärverschlusselement 26 CP 1 M 75 FHL6, Koester GmbH & Co. KG Deutschland, Industriestraße 2, 96146 Altendorf, Germany.

Verschlusselemente 40, 42 an den Latzabschnitten 32, 34: CP 1 M 45 NC FHL1, Koester GmbH & Co. KG Deutschland, Industriestraße 2, 96146 Altendorf, Germany.

Weiterhin können in der Fixier- und Tragevorrichtung elastische Bereich vorhanden sein, die beispielsweise aus einem elastischen Laminat FlexEar 80 PA 70 V2 EMB, der Firma Koester GmbH & Co. KG Deutschland, Industriestraße 2, 96146 Altendorf, Germany, gebildet werden.

### Test zur Ermittlung der Scherkräfte

Die Ermittlung der Scherkräfte erfolgt gemäß dem in EP-1655006-B1 beschriebenen Verfahren an einer gekrümmten Oberfläche, die die Bauchrundung simuliert: "Abzug über Bauch".

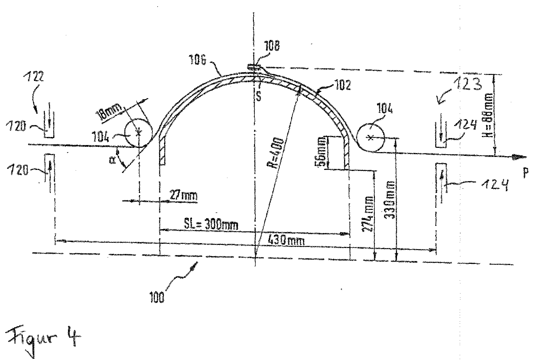

Für die Durchführung der Prüfmethode kann ein Zugprüfgerät Typ Z010 / TN 2S, Messdose 100 N, erhältlich bei der Firma Zwick GmbH & Co KG, Ulm, Deutschland, mit einer Klemmbackenbreite zum Einspannen des Prüflings von 60 mm verwendet werden. Bei der Durchführung der Prüfmethode wird das zu prüfende mechanisch wirkende Verschlusssystem aus einer hakenbildenden Komponente und einer schlaufenbildenden Komponente über eine gekrümmte Fläche gelegt, welche eine Rundung des Bauchbereichs eines Benutzers simulieren soll (siehe Figur 4). Zur Verbindung der hakenbildenden und der schlaufenbildenden Verschlusskomponenten mit den Klemmbacken des Zugprüfgeräts wird ein biegsames Substrat, beispielsweise ein einseitig klebendes Klebeband einer bevorzugten Breite von 25 mm, bzw. 50 mm, das unter der Bezeichnung STA 306 bei der Firma 3M Deutschland GmbH ansässig in Neuss erhältlich ist, verwendet. Das Klebeband ist aus Polypropylen, seine Oberfläche ist beschichtet durch ein urethanmodifiziertes Siliconpolymer. Das Flächengewicht des Haftklebstoffauftrags beträgt 23 g/m2. Der über die gekrümmte Fläche gelegte Prüfling, bestehend aus aufeinander haftenden flächenhaften Abschnitten des Verschlusssystems, wird durch Verwendung des Zugprüfgeräts auf Zug beansprucht, woraus sich eine scherende Beanspruchung der aneinander haftenden flächenhaften Abschnitte ergibt.

### Probenvorbereitung:

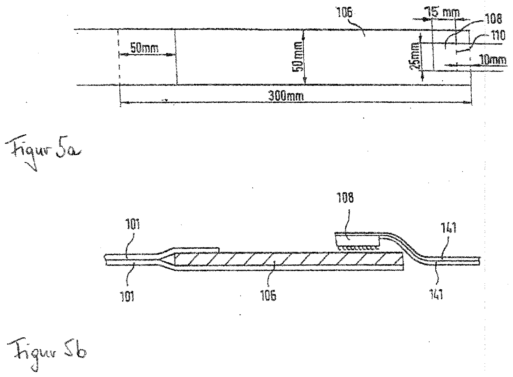

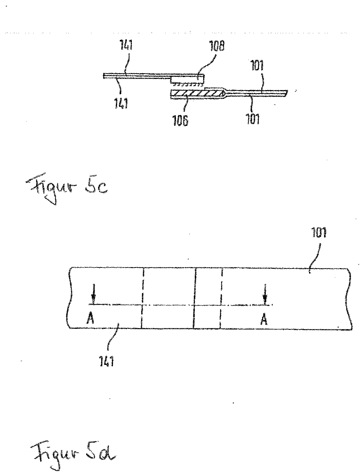

Die zu verwendenden mechanischen Verschlusselemente, also das Material der schlaufenbildenden Komponente (106), das die Außenseite des Hüftgürtels 6 bildet, und das Material der hakenbildenden Komponente (108), das die Gürtelverschlusselemente (22), Sekundärverschlusselemente (26) und Verschlusselemente (40, 42) am Latzabschnitt (32, 34) bildet, werden über 24 h bei 23° C und 50 % relativer Luftfeuchte konditioniert. Es werden Prüflinge einer Größe von 50 × 300 mm aus der schlaufenbildenden Komponente ausgestanzt und sandwichartig mittig durch zwei mit ihren Klebeflächen gegeneinander geklebte einseitige Klebebänder 101 einer Breite von 50 mm derart fixiert, dass das untere Klebeband die Rückseite des flächenhaften Abschnittes der schlaufenbildenden Komponente überfängt und das obere Klebeband die obere Seite (bei einem Vliesfolienlaminat ist die obere Seite die Vliesseite) der schlaufenbildenden Komponente in der Länge um 50 mm einfasst, so dass ein Überstand der schlaufenbildenden Komponente von 50 × 250 mm resultiert (siehe Figur 5a, Figur 5b). Desgleichen wird die hakenbildende Komponente des mechanischen Verschlusselements 108 in einer Länge von 15 mm und in einer Breite von 25 mm ausgestanzt und durch zwei mit ihren Klebeflächen gegeneinander geklebten einseitigen Klebebändern 141 einer Breite von 25 mm derart fixiert, dass das obere Klebeband die Rückseite des flächenhaften Abschnittes der hakenbildenden Komponente überfängt und das untere Klebeband bündig an den flächenhaften Abschnitt der hakenbildenden Komponente angrenzt (siehe Figur 5a und Figur 5b). Der flächenhafte Abschnitt der hakenbildenden Komponente des mechanischen Verschlusselements 108 wird nun auf die schlaufenbildende Komponente 106 aufgelegt, wobei der Abstand von der Längsendkante der Schlaufen-Komponente 10 mm und von den seitlichen Längsrändern je 12,5 mm betragen soll (s. Figur 5a). Wenn die zur Verfügung stehende schlaufenbildende Komponente 106 von vornherein eine kleinere Abmessung aufweist, so dass keine Bereitstellung eines 50 mm × 300 mm großen Prüfling möglich ist, wird die Größe des Prüflings mit einer Breite von 25 mm und einer derart bemessenen Länge gewählt und dieser Abschnitt sandwichartig mittig zwischen die Enden zweier einseitiger oben näher gekennzeichneter Klebebänder 101 einer Breite von 25 mm derart fixiert, dass das untere Klebeband die Rückseite des flächenhaften Abschnittes der schlaufenbildenden Komponente überfängt und das obere Klebeband die obere Seite der schlaufenbildenden Komponente in der Länge derart einfasst, dass die Länge des Überstandes der schlaufenbildenden Komponente der Länge des Abschnittes des hakenbildende Komponente des mechanischen Verschlusselements 108 entspricht (siehe Figur 5c, Figur 5d). Solchenfalls werden die so vorbereiteten Abschnitte der mechanischen Verschlusselemente, also das Material der schlaufenbildenden Komponente (106) und das Material der hakenbildenden Komponente (108) vollflächig übereinander gelegt (Figur 5c, Figur 5d).

Sollte kein 25 mm breiter Abschnitt der mechanischen Verschlusselemente 108 zur Verfügung stehen, wird ein entsprechend schmalerer Abschnitt verwendet. Solchenfalls werden die ermittelten Kraftwerte rechnerisch auf einen 25 mm breiten Abschnitt normiert, derart dass die gemessenen Kräfte mit einem Faktor f multipliziert werden, welcher sich ergibt aus , wobei x die Breite des Prüflings gemessen in mm ist.

Die so oder auf die zuvor beschriebene Art aufeinander gelegten flächenhaften Abschnitte werden durch viermaliges Anrollen mit einer 50 mm breiten und im Durchmesser 100 mm starken Rolle mit glatter Oberfläche und einem Rollengewicht von 5 kg miteinander verbunden, wobei die Anrollgeschwindigkeit 20-100 mm/sec beträgt.

### Prüfverfahren:

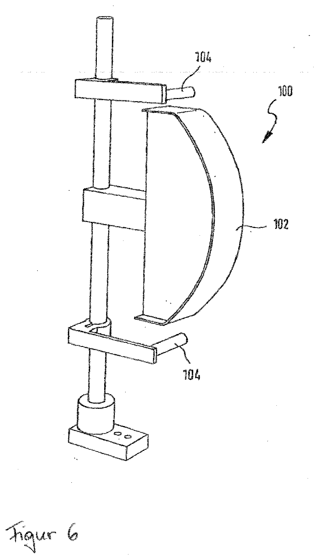

Die wie vorstehend beschrieben verlängerte schlaufenbildende Komponente 106 wird in die untere Klemmbacke 122 des Zugprüfgeräts mittig zentriert eingespannt, und das gegenüberliegende Ende der wie vorstehend beschriebenen verlängerten hakenbildenden Komponente 108 wird in die bewegliche obere Klemmbacke 123 des Zugprüfgeräts ebenfalls zentriert eingespannt. Der so eingespannte Prüfling wird über die aus Figur 4, Figur 6 ersichtliche Vorrichtung 100, welche den Bauch- oder Hüftbereich eines Benutzers simulieren soll, gelegt. Diese Vorrichtung 100 ist in Figur 6 perspektivisch dargestellt. Man erkennt eine bogenförmig gekrümmte Fläche 102 aus poliertem Stahl mit einer Rautiefe von 5 bis 25 mm und mit einem Krümmungsradius R von zumindest abschnittsweise 400 mm und einer Sehnenlänge SL von 300 mm. Des Weiteren sind oberhalb und unterhalb der gekrümmten Fläche 102 Umlenkrollen 104 mit einem Durchmesser von 18 mm vorgesehen, welche den über die gekrümmte Fläche gelegten Prüfling in die vertikale Richtung um H = 88 mm umlenken, wo er dann mit Klemmen 120, 124 des nicht dargestellten Zugprüfgeräts verbunden wird. Die Umlenkung erfolgt um einen Winkel α von 60°. Hierdurch wird der Abzugswinkel im Wesentlichen tangential zu der gekrümmten Fläche und konstant gehalten. Die aufeinander gelegten flächenhaften Abschnitte 106, 108 der Komponenten der Verschlusselemente werden in Bezug auf die gekrümmte Fläche 102 so positioniert, dass die haken- und schlaufenbildenden Komponenten des mechanischen Verschlusselements mittig zentriert im Scheitelpunkt S der gekrümmten Fläche 102 zu liegen kommen. Es wird dann die bewegliche Klemmbacke 124, mit der die haken- und schlaufenbildenden Komponenten des mechanischen Verschlusselements verbunden ist, mit der nachfolgend angegebenen Prüfgeschwindigkeit in Pfeilrichtung P bewegt, und es wird währenddessen die dabei auftretende Zugkraft zwischen den Klemmen ermittelt. Die Prüfparameter sind:
- Prüfgeschwindigkeit: 300 mm/min
- Einspannlänge des Prüflings: 430 mm (s. Figur 4)
- Messweg: Strecke bis zur Ablösung der Verschlussmittelkomponenten voneinander
- Vorkraft: 0,2 N
- Prüfanzahl: n ≥ 5.

Die Auswertung erfolgt dergestalt, dass die bis zum voneinander Ablösen der Verschlusselemente ermittelte Maximalkraft gerundet auf zwei Dezimalstellen in N (Newton) notiert wird und in Form eines Mittelwerts der n-Messungen angegeben wird.

Tabelle 1 zeigt die Scherkräfte bei Abzug der hakenbildenden Komponente über eine auf eine Rundung aufgebrachte schlaufenbildende Komponente in Newton [N] pro 25 mm hakenbildende Komponente.

### Tabelle 1 [N/25mm]

**Tabelle 1**

| Messung Nr. | M1 / GI | | M1 / GA | | M2 / GA | | M2 / GI | | Backsheet der Vorlage | |
|---|---|---|---|---|---|---|---|---|---|---|
| | quer | längs | quer | längs | quer | längs | quer | längs | quer | längs |
| 1 | 5,21 | 1,93 | 34,00 | 24,99 | 24,57 | 16,12 | 30,98 | 22,05 | 2,97 | 1,76 |
| 2 | 10,49 | 0,78 | 30,28 | 30,07 | 35,01 | 24,83 | 30,96 | 11,24 | 1,94 | 1,39 |
| 3 | 9,86 | 1,66 | 30,51 | 27,45 | 30,77 | 31,80 | 36,50 | 12,57 | 2,83 | 2,32 |
| 4 | 12,24 | 1,06 | 31,15 | 33,65 | 31,02 | 9,24 | 28,73 | 14,82 | 5,29 | 2,12 |
| 5 | 8,84 | 3,78 | 31,80 | 28,00 | 21,37 | 17,50 | 35,26 | 23,64 | 4,69 | 4,85 |
| | | | | | | | | | | |
| Mittelwert | **9,3** | **1,8** | **31,5** | **28,8** | **28,5** | **19,9** | **32,5** | **16,9** | **3,5** | **2,5** |

| | | | | | | | | | | |
|---|---|---|---|---|---|---|---|---|---|---|
| Legende M1 / GI: Material 1 der Fixier- und Tragevorrichtung (Hüftgürtelinnenseite; Spinnvlies-Seite) M1 / GA: Material 1 der Fixier- und Tragevorrichtung (Hüftgürtelaußenseite; Kardenvlies-Seite) M2 / GA: Material 2 der Fixier- und Tragevorrichtung (Hüftgürtelaußenseite) M2 / GI: Material 2 der Fixier- und Tragevorrichtung (Hüftgürtelinnenseite) | | | | | | | | | | |

Die Scherkräfte des Materials M1 sind auf der Hüftgürtelinnenseite GI und auf der Hüftgürtelaußenseite GA des Materials signifikant unterschiedlich. Dies hat den Vorteil, dass sich die Vorlagen-Hakenkomponente leicht von der Hüftgürtelinnenseite lösen lassen, wenn der Hüftgürtel entfaltet und angelegt wird.

Die Scherkräfte des alternativen Materials M2 sind auf der Hüftgürtelinnenseite GI und Hüftgürtelaußenseite GA des Materials im Rahmen der Messgenauigkeit gleich.

Die Scherkräfte des Backsheets der Vorlage sind in Längsrichtung um den Faktor 5 bis 12 niedriger, als die Scherkräfte der Hüftgürtelaussenseiten GA der Materialien M1 und M2 und im Rahmen der Messgenauigkeit vergleichbar mit den Scherkräften der Verschlusselemente auf der Hüftgürtelinnenseite des Materials M1. In Querrichtung sind die Scherkräfte des Backsheets der Vorlage um den Faktor 7,5 bis 11 niedriger, als die Scherkräfte der Hüftgürtelaussenseiten GA der Materialien M1 und M2 und um den Faktor 2 kleiner als die Scherkräften der Verschlusselemente auf der Hüftgürtelinnenseite des Materials M1.

Die Scherkraft des Backsheets der Vorlage in Materiallängsrichtung von 2,5 N/25 mm entspricht bei einer Abschnittslänge der Verschlusselemente der Latzabschnitte von durchschnittlich 11 cm (11,5 - 10,5 cm bei trapezförmigem Zuschnitt) einer Haftkraft (Scherkraft über Bauch) dieser Verschlusselemente von 11 N (längs) und analog bei 3,5 N/25mm von 15,4 N (quer).

### Test zur Ermittlung der Peelkraft

Die im folgenden beschriebene Methode zur Bestimmung von Peelkräften mechanischer Verschlusselemente zeichnet sich dadurch aus, dass die hakenbildende Komponente fixiert ist und die schlaufenbildende Komponente abgezogen wird. Dies ist vorteilhaft, wenn die Starrheit der hakenbildende Komponente einen kontinuierlichen Abzug nicht erlaubt, insbesondere nicht bei einem Abzugwinkel von 180°.

### Probenvorbereitung

Die zu untersuchenden Materialien werden vor der Prüfung 24 h im Normklima gelagert (23 °C, 50 % rF) und die Prüfung erfolgt ebenfalls unter diesen Bedingungen. Eine Stahlplatte, 50 mm breit, wird mittels eines doppelseitigen Klebebandes, 50 mm breit (Fa. 3M, Typ 410) mit einem druckelastischen Material, erhältlich unter der Bezeichnung Lastocomb (No. 931857/0; PZN - 0614191, Fa. Paul Hartmann AG, EAN 4049500927291), 50 mm breit, 100 mm lang, abgedeckt. Die hakenbildende Komponente des Verschlusselements mit einer Länge von 10 cm wird in Längsrichtung mittig auf dem Lastocomb mit doppelseitigem Klebeband angebracht und zweimal mit einem automatischen Überroller mit einem Gewicht von 2 kg angerollt. Es wird darauf geachtet, dass das doppelseitige Klebeband vollständig durch die Materialien (Lastocomp und hakenbildende Komponente des Verschlusselements) abgedeckt ist und seinerseits die Prüfung nicht beeinflusst. Außerdem werden alle Materialien blasen- und faltenfrei auf die Stahlplatte aufgebracht.

Auf derart vorbereitete Stahlplatten mit hakenbildenden Verschlusselementen werden jeweils die schlaufenbildenden Komponenten, das heisst, die Außenseite (Backsheet) der Inkontinenzvorlage und die Außen- und Innenseite der Fixier- und Tragvorrichtung (je 50 mm breit, je 300 mm lang) mittig in Längsrichtung der Materialien der schlaufenbildenden Komponenten auf das hakenbildende Verschlusselemente gelegt, so dass das hakenbildende Verschlusselemente vollständig bedeckt ist und mit einer Rolle mit glatter Oberfläche mit einem Rollengewicht von 5 kg viermal angerollt.

### Prüfverfahren

Zur Durchführung der Prüfung wird eine Werkstoffprüfmaschine nach DIN EN ISO 75001-1 verwendet. Die Stahlplatte wird am freien Ende in die feststehende Klemme der Prüfmaschine eingespannt, das Material der schlaufenbildenden Komponente wird in die bewegliche Klemme gespannt und in einem Winkel von 180° von dem Hakenverschlusselement mit einer Abzugsgeschwindigkeit von 300 mm/min abgezogen. Die Peelkraft wird ohne Einhalten einer Wartezeit ermittelt. Die Prüfparameter sind:
Einspannlänge: 149 mm
Vormessweg: 10 mm
Messweg 120 mm
Nachmessweg: 10 mm
Prüfgeschwindigkeit: 300 mm/min
Probenbreite: 25 mm
Spitzendefinition: 0,2 N
Vorkraft: 0,1 N

Es werden jeweils 5 Proben vermessen und die mittlere Kraft während des Messweges, das heißt, die Strecke bis zur Ablösung der Verschlussmittelkomponenten voneinander, bestimmt. Die Auswertung erfolgt nach DIN ISO 6133, Verfahren B (automatische Spitzenwertberechnung). Angegeben werden die Mittelwerte in Newton [N] bezogen auf die Probenbreite in [mm], gerundet auf eine Dezimalstelle.

Tabelle 2 zeigt die Peelkräfte in Newton [N]/ 15 mm bei Abzug der schlaufenbildenden Komponente von einer hakenbildenden Komponente bei einem Abzugwinkel von 180°(bezogen auf die aktive Breite der hakenbildenden Komponente von 15 mm).

### Tabelle 2: [N]/15 mm

**Tabelle 2**

| Messung Nr. | M1 / GI | M1 / GA | | M2 / GA und GI | | Backsheet der Vorlage | |
|---|---|---|---|---|---|---|---|
| | quer | längs | quer | längs | quer | längs | quer |
| 1 | 0,11 | 0,53 | 0,90 | 0,89 | > 8* | 0,35 | 0,18 |
| 2 | 0,07 | 0,39 | 0,61 | 1,26 | > 8* | 0,27 | 0,22 |
| 3 | 0,11 | 0,87 | 0,77 | 0,85 | > 8* | 0,15 | 0,32 |
| 4 | 0,15 | 0,78 | 0,67 | 0,63 | > 8* | 0,21 | 0,34 |
| 5 | 0,11 | 0,60 | 1,08 | 0,8 | > 8* | 0,21 | 0,31 |
| 6 | 0,13 | 1,03 | 0,44 | 0,5 | > 8* | 0,16 | 0,17 |
| Mittelwert | **0,11** | **0,70** | **0,75** | **0,88** | **> 8*** | **0,23** | **0,26** |

| | | | | | | | |
|---|---|---|---|---|---|---|---|
| Legende M1 / GI: Material 1 der Fixier- und Tragevorrichtung (Hüftgürtelinnenseite; Spinnvlies-Seite) M1 / GA: Material 1 der Fixier- und Tragevorrichtung (Hüftgürtelaußenseite; Kardenvlies-Seite) M2 / GA: Material 2 der Fixier- und Tragevorrichtung (Hüftgürtelaußenseite) M2 / GI: Material 2 der Fixier- und Tragevorrichtung (Hüftgürtelinnenseite) * Werte außerhalb des Messbereichs | | | | | | | |

Die Peelkräfte des Materials M1 sind auf der Hüftgürtelinnenseite GI (quer) und auf der Hüftgürtelaußenseite GA (längs und quer) des Materials signifikant unterschiedlich. Dies hat den Vorteil, dass sich die Vorlagen-Hakenkomponente leicht von der Hüftgürtelinnenseite lösen lassen, wenn der Hüftgürtel entfaltet und angelegt wird. Die Peelkräfte des Materials M1 auf der Hüftgürtelinnenseite GI (längs) sind aufgrund der Materialeigenschaften des thermolaminierten Spinnvlieses so niedrig, dass keine relevante Haftung der hakenbildenden Komponente erfolgt.

Die Peelkräfte des alternativen Materials M2 sind auf der Hüftgürtelinnenseite GI und Hüftgürtelaußenseite GA des Materials identisch. Bei Messung in Querrichtung liegen die Werte mit > 8 N außerhalb des Messbereiches, da es zu einer Dehnung des Vlieses kommt, wodurch die Haken der hakenbildendenden Komponente sich in das Material stärker "einarbeiten", dass heisst, es entstehen durch die Dehnung Poren, in die sich die Haken stärker verhaken können.

Die Peelkräfte des Backsheets der Vorlage sind bei Messung in Längs- und Querrichtung signifikant niedriger, als die Peelkräfte auf den Hüftgürtelaußenseiten GA der Materialien M1 und M2 in Längs- und Querrichtung. Dies ist von praktischer Relevanz beim Wechseln der Vorlage. Die Vorlagen-Hakenkomponente lassen sich unproblematisch öffnen und wieder auf einer neuen Vorlage schließen, während die Hüftgürtel-Hakenkomponente aufgrund der höheren Peelkräfte unverändert in ihrer Position bleiben.

Test zur Überprüfung der sicheren Fixierung der Verschlusselemente der Latzabschnitte auf der Inkontinenzvorlage unter hoher Gewichtslast

Aus der Fixier- und Tragevorrichtung wird jeweils ein Materialabschnitt aus dem Bauchbereich (14) und dem Rückenbereich (16) entnommen. Die beiden Materialabschnitte umfassen die Verschlusselemente (40, 42) auf den Latzabschnitten (32, 34), sowie die Teilabschnitte vom Bauch- und Rückenbereich, die sich von den Verschlusselementen (40, 42) bis zum oberen Rand (proximale Querkante) der Fixier- und Tragevorrichtung erstrecken und zwar in der Breite der Verschlusselemente (40, 42), die in diesem Fall 11,5 cm beträgt.

Diese Materialabschnitte werden analog zur vorgesehenen Tragesituation der Fixier- und Tragevorrrichtung an der Inkontinenzvorlage fixiert. Das heisst, dass die Verschlusselemente (40, 42) auf der Aussenseite (Backsheet) der Inkontinenzvorlage fixiert werden und zwar so, dass in Längsrichtung die vordere und hintere Querkante der Inkontinenzvorlage mit dem oberen Rand der Materialabschnitte übereinstimmt und dass in Querrichtung die Verschlusselemente mittig auf der Inkontinenzvorlage positioniert sind. Dann wird mit einem 5kg Rolle mit glatter Oberfläche 4 mal über die Verschlusselemente auf der Inkontinenzvorlage gerollt.

Die Vorlage wird auf ein Zylinderrohr mit einem Durchmesser von 24 cm und einer Höhe von 16 cm von außen an dem Rohr fixiert, so dass die vordere und hintere Querkante der Inkontinenzvorlage und der obere, Rand der Materialabschnitte am oberen Rand des Zylinderrohres zu liegen kommen und sich gegenüber liegen. Dazwischen befindet sich der Schrittbereich der Inkontinenzvorlage, in Analogie zur Tragesituation. Die Fixierung erfolgt mit einem Klebeband ausreichender Haftkraft auf der Aussenseite der Fixier- und Tragevorrichtung in der Form, dass die Inkontinenzvorlage selbst keinen Kontakt mit dem Klebeband besitzt und der jeweilige obere Rand der Materialabschnitte mit dem oberen Rand des Zylinders abschließt und das Klebeband von der Aussenseite der Inkontinenzvorlage über den Rand des Zylinders auf die Innenseite des Zylinders geführt wird. Durch diese Anordnung ist sichergestellt, dass die Gewichtskräfte, die im nachfolgenden Versuch auf die Verschlusselemente durch die beladene Inkontinenzvorlage wirken, nicht ganz oder zum Teil durch die Klebebänder gehalten werden, sondern ausschließlich durch die Verschlusselemente.

Der Zylinder mit der Inkontinenzvorlage wird senkrecht zur Achse positioniert, wobei die Vorlage frei nach unten hängt. Die Inkontinenzvorlage wird mit 1000 ml einer 0,9% Kochsalzlösung innerhalb von 10 min von oben beladen.

Die Inkontinenzvorlage wird dabei ausschließlich durch die Verschlusselemente gehalten, die die durch die Beladungsmenge erzeugte Gewichtskraft von 9,81 N, das heisst, von 4,9 N je Verschlusselement zzgl. des Eigengewichts der Inkontinenzvorlage von ca. 1 N sicher halten über einen Zeitraum von mindestens 10 min.

Diese Anordnung hat gegenüber einer Anordnung, in der statt der beiden Materialabschnitte der Fixier- und Tragevorrichtung die gesamte Vorrichtung verwendet wird, den Vorteil, dass keine zusätzliche Zugspannung in Hüftumfangsrichtung (Zylinderumfangsrichtung), die insbesondere durch die elastischen Abschnitte der Fixier- und Tragevorrichtung hervorgerufen würde, herrscht.

## Patentansprüche

1. Fixier- und Tragevorrichtung (2) für eine wegwerfbare absorbierende Inkontinenzvorlage (4) und Inkontinenzvorlage, wobei die Fixier- und Tragevorrichtung (2) mit einem mittels Gürtelverschlusselementen (22, 24, 25) auf sich selbst lösbar schließbaren und so eine in Hüftumfangsrichtung (8) durchgehende Hüftöffnung bildenden Hüftgürtel (6) an dem die Inkontinenzvorlage (4) lösbar fixierbar ist, so dass sie im Schrittbereich des Benutzers getragen und nach Gebrauch wieder vom Hüftgürtel (6) entfernt und verworfen werden kann, wobei der Hüftgürtel (6) einen vorderen Bauchbereich (14), einen hinteren Rückenbereich (16) und einen linken und einen rechten Seitenbereich (18, 20) umfasst, und der Hüftgürtel (6), ausgehend von dem Rückenbereich (16) und dem Bauchbereich (14) je einen sich in einer Längsrichtung in Richtung (38) auf den Schrittbereich des Benutzers erstreckenden Latzabschnitt (32, 34) aufweist, der an seiner körperzugewandten Seite Verschlusselemente (40, 42) aufweist, die mit komplementären Verschlusselementen auf der körperabgewandten Seite der Inkontinenzvorlage (4) lösbar haftend zusammenwirken, **dadurch gekennzeichnet, dass** die aufeinander fixierten Gürtelverschlusselemente (22) bei angelegter Fixier- und Tragevorrichtung (2) eine größere oder gleiche Haltekraft aufweisen, als die am Latzabschnitt (32, 34) angeordneten Verschlusselemente (40, 42) auf den komplementären Verschlusselementen der Inkontinenzvorlage (4).

2. Fixier- und Tragevorrichtung nach Anspruch 1, **dadurch gekennzeichnet, dass** die Verschlusselemente (40, 42) des Latzabschnitts (32, 34) vor der Ingebrauchnahme auf der körperzugewandten Seite des Latzabschnitts und/oder auf den Bauch- und Rückenbereich (14,16) des Hüftgürtel (6) eingeschlagen und in dieser Konfiguration leicht lösbar an der körperzugewandten Seite des Latzabschnitts (32, 34) und/oder des Bauch- und Rückenbereich (14,16) des Hüftgürtels (6) haftend sind.

3. Fixier- und Tragevorrichtung nach Anspruch 2, **dadurch gekennzeichnet, dass** die Haltekräfte der Gürtelverschlusselemente (22, 24, 25) aufeinander größer oder gleich den Haltekräften der Verschlusselemente (40, 42) der Latzabschnitte (32, 34) auf den Latzabschnitten (32, 34) bzw. dem Bauch- und Rückenbereich (14,16) des Hüftgürtels (6) sind.

4. Fixier- und Tragevorrichtung nach einem oder mehreren der vorangehenden Ansprüche, **dadurch gekennzeichnet, dass** die Haltekraft der Verschlusselemente (40, 42) der Latzabschnitte (32, 34) auf den komplementären Verschlusselementen der Inkontinenzvorlage (4) größer als die Gewichtskraft der Inkontinenzvorlage (4) in einem definiert maximalen Beladungszustand ist.

5. Fixier- und Tragevorrichtung nach Anspruch 4, **dadurch gekennzeichnet, dass** die Gewichtskraft in einem maximalen Beladungszustand durch einen produktbezogenen "absorption before leakage"-Wert gemäß EDANA-Prüfmethode WSP 354.0 definiert ist.

6. Fixier- und Tragevorrichtung nach Anspruch 4 oder 5, **dadurch gekennzeichnet, dass** die Gewichtskraft in einem maximalen Beladungszustand zwischen 2 und 15 N, vorzugsweise zwischen 2,5 und 12 N, weiter bevorzugt zwischen 3 und 11 N liegt.

7. Fixier- und Tragevorrichtung nach einem oder mehreren der vorangehenden Ansprüche, **dadurch gekennzeichnet, dass** sich der Latzabschnitt (32, 34) in Richtung (38) auf den Schrittbereich verjüngt.

8. Fixier- und Tragevorrichtung nach einem oder mehreren der vorangehenden Ansprüche, **dadurch gekennzeichnet, dass** die Verschlusselemente (40, 42) des Latzabschnitts zumindest an einem schrittzugewandten Ende des Latzabschnitts vorgesehen sind.

9. Fixier- oder Tragevorrichtung nach einem oder mehreren der vorangehenden Ansprüche, **dadurch gekennzeichnet, dass** die Verschlusselemente (40, 42) des Latzabschnitts (32, 34) auf einem streifenförmigen und quer zur Längsrichtung erstreckten Materialabschnitt vorgesehen sind, der an den Latzabschnitt (32, 34) angefügt ist.

10. Fixier- und Tragevorrichtung nach einem oder mehreren der vorangehenden Ansprüche, **dadurch gekennzeichnet, dass** die Verschlusselemente des Latzabschnitts (32, 34) und/oder die Gürtelverschlusselemente (22) und/oder die Sekundärverschlusselemente (26) mechanisch und/oder klebend wirkende Verschlusselemente sind.

11. Fixier- und Tragevorrichtung nach einem oder mehreren der vorangehenden Ansprüche, **dadurch gekennzeichnet, dass** der Hüftgürtel (6) nur in einem Bereich öffenbar und schließbar ist und die Gürtelverschlusselemente (22, 24, 25) in diesem Bereich an freien Endabschnitten (10, 12) des Hüftgürtels (6) vorgesehen sind.

12. Fixier- und Tragevorrichtung nach einem oder mehreren der vorangegangenen Ansprüche, **dadurch gekennzeichnet, dass** Sekundärverschlusselemente (26) vorgesehen sind, mittels derer eine Umfangslänge des Hüftgürtels einstellbar und so zumindest der vordere Latzabschnitt (32) symmetrisch zum Schritt des Benutzers positionierbar ist.

13. Fixier- oder Tragevorrichtung nach einem oder mehreren der vorangegangenen Ansprüche, **dadurch gekennzeichnet, dass** die Gürtelverschlusselemente (22, 24, 25) und die Sekundärverschlusselemente (26) beidseits des Bauchbereichs (14) oder in Seitenbereichen (18, 20) des Hüftgürtels (6) vorgesehen sind.

14. Fixier- oder Tragevorrichtung nach einem oder mehreren der vorangehenden Ansprüche, **dadurch gekennzeichnet, dass** der Bauchbereich (14) und/oder der Rückenbereich (16) des Hüftgürtels (6) von einem Vliesmaterial oder Vliesverbundmaterial gebildet ist.

## Claims

1. A fastening and supporting device (2) for a disposable absorbent incontinence pad (4), and an incontinence pad, the fastening and supporting device (2) having a hip belt (6), which is detachably closable on itself by means of belt closure elements (22, 24, 25) and which thus forms a continuous hip opening in the circumferential hip direction (8), to which belt the incontinence pad (4) is detachably fastenable, such that the incontinence pad can be worn in the crotch area of the user and removed from the hip belt (6) and discarded after use, the hip belt (6) comprising a front stomach area (14), a rear back area (16), and a left and a right side area (18, 20), and the hip belt (6), starting from the back area (16) and the stomach area (14), having in each case one flap portion (32, 34) extending in a longitudinal direction in the direction (38) of the crotch area of the user and having closure elements (40, 42) on its side facing the body that interact in a detachably adherent manner with complementary closure elements on the side of the incontinence pad (4) facing away from the body, **characterized in that** when the fastening and supporting device (2) is attached, the belt closure elements (22) that are fastened to one another have a retaining force that is greater than or equal to the retaining force of the closure elements (40, 42), situated on the flap portion (32, 34), on the complementary closure elements of the incontinence pad (4).

2. The fastening and supporting device according to Claim 1, **characterized in that**, prior to use, the closure elements (40, 42) of the flap portion (32, 34) are folded in on the side of the flap portion facing the body and/or onto the stomach and back areas (14, 16) of the hip belt (6), and in this configuration are adherent in an easily detachable manner on the side of the flap portion (32, 34) facing the body and/or the stomach and back areas (14, 16) of the hip belt (6).

3. The fastening and supporting device according to Claim 2, **characterized in that** the retaining forces of the belt closure elements (22, 24, 25) on one another are greater than or equal to the retaining forces of the closure elements (40, 42) of the flap portions (32, 34) on the flap portions (32, 34) or on the stomach and back areas (14, 16) of the hip belt (6).

4. The fastening and supporting device according to one or more of the preceding claims, **characterized in that** the retaining force of the closure elements (40, 42) of the flap portions (32, 34) on the complementary closure elements of the incontinence pad (4) is greater than the weight force of the incontinence pad (4) in a defined maximum load state.

5. The fastening and supporting device according to Claim 4, **characterized in that** the weight force in a maximum load state is defined by a product-related "absorption before leakage" value according to EDANA test method WSP 354.0.

6. The fastening and supporting device according to Claim 4 or 5, **characterized in that** the weight force in a maximum load state is between 2 and 15 N, preferably between 2.5 and 12 N, more preferably between 3 and 11 N.

7. The fastening and supporting device according to one or more of the preceding claims, **characterized in that** the flap portion (32, 34) tapers in the direction (38) of the crotch area.

8. The fastening and supporting device according to one or more of the preceding claims, **characterized in that** the closure elements (40, 42) of the flap portion are provided at least at one end of the flap portion facing the crotch.

9. The fastening or supporting device according to one or more of the preceding claims, **characterized in that** the closure elements (40, 42) of the flap portion (32, 34) are provided on a strip-shaped material portion which extends transversely with respect to the longitudinal direction and is attached to the flap portion (32, 34).

10. The fastening and supporting device according to one or more of the preceding claims, **characterized in that** the closure elements of the flap portion (32, 34) and/or the belt closure elements (22) and/or the secondary closure elements (26) are mechanically and/or adhesively acting closure elements.

11. The fastening and supporting device according to one or more of the preceding claims, **characterized in that** the hip belt (6) is openable and closable only in one area, and the belt closure elements (22, 24, 25) in this area are provided at free end portions (10, 12) of the hip belt (6).

12. The fastening or supporting device according to one or more of the preceding claims, **characterized in that** secondary closure elements (26) are provided by means of which a circumferential length of the hip belt is adjustable, and thus at least the front flap portion (32) is positionable symmetrically with respect to the crotch of the user.

13. The fastening or supporting device according to one or more of the preceding claims, **characterized in that** the belt closure elements (22, 24, 25) and the secondary closure elements (26) are provided on either side of the stomach area (14) or in side areas (18, 20) of the hip belt (6).

14. The fastening or supporting device according to one or more of the preceding claims, **characterized in that** the stomach area (14) and/or the back area (16) of the hip belt (6) are/is formed by a nonwoven material or nonwoven composite material.

## Revendications

1. Dispositif de fixation et de support (2) pour une protection d'incontinence (4) absorbante jetable et protection d'incontinence, dans lequel ledit dispositif de fixation et de support (2) comprend une ceinture de hanches (6) qui peut être fermée sur elle-même de manière amovible au moyen d'éléments de fermeture de ceinture (22, 24, 25) et forme ainsi une ouverture de hanches continue dans la direction circonférentielle des hanches (8) et sur laquelle la protection d'incontinence (4) peut être fixée de manière amovible de sorte qu'elle peut être portée dans la zone d'entrejambe de l'utilisateur et peut, après l'usage, être détachée à nouveau de la ceinture de hanches (6) et être jetée, ladite ceinture de hanches (6) comprenant une zone ventrale avant (14), une zone dorsale arrière (16) et des zones latérales gauche et droite (18, 20), et ladite ceinture de hanches (6) présentant, à partir de ladite zone dorsale (16) et de ladite zone ventrale (14), respectivement une portion bavette (32, 34) qui s'étend dans une direction longitudinale vers (38) ladite zone d'entrejambe de l'utilisateur et qui présente, sur sa face tournée vers le corps, des éléments de fermeture (40, 42) qui coopèrent par adhérence amovible avec des éléments de fermeture complémentaires présents sur la face de la protection d'incontinence (4), qui est opposée au corps, **caractérisé par le fait que**, lorsque le dispositif de fixation et de support (2) est appliqué, les éléments de fermeture de ceinture (22) fixés les uns aux autres présentent une force de maintien supérieure ou égale à celle que présentent les éléments de fermeture (40, 42) disposés sur la portion bavette (32, 34), sur les éléments de fermeture complémentaires de la protection d'incontinence (4).

2. Dispositif de fixation et de support selon la revendication 1, **caractérisé par le fait que**, avant la mise en usage, les éléments de fermeture (40, 42) de la portion bavette (32, 34) sont rabattus sur la face de la portion bavette qui est tournée vers le corps et/ou sur la zone ventrale et dorsale (14, 16) de la ceinture de hanches (6), et que, dans cette configuration, ils adhèrent de manière à être facilement détachables sur la face tournée vers le corps de la portion bavette (32, 34) et/ou de la zone ventrale et dorsale (14, 16) de la ceinture de hanches (6).

3. Dispositif de fixation et de support selon la revendication 2, **caractérisé par le fait que** les forces de maintien des éléments de fermeture de ceinture (22, 24, 25) les uns sur les autres sont supérieures ou égales aux forces de maintien des éléments de fermeture (40, 42) des portions bavettes (32, 34) sur les portions bavettes (32, 34) ou bien la zone ventrale et dorsale (14, 16) de la ceinture de hanches (6).

4. Dispositif de fixation et de support selon l'une ou plusieurs des revendications précédentes, **caractérisé par le fait que** la force de maintien des éléments de fermeture (40, 42) des portions bavettes (32, 34) sur les éléments de fermeture complémentaires de la protection d'incontinence (4) est supérieure au poids de la protection d'incontinence (4) dans un état de charge maximal défini.

5. Dispositif de fixation et de support selon la revendication 4, **caractérisé par le fait que** le poids dans un état de charge maximal est défini par une valeur « absorption avant fuite » relative au produit, selon la méthode d'essai EDANA WSP 354.0.

6. Dispositif de fixation et de support selon la revendication 4 ou 5, **caractérisé par le fait que** le poids dans un état de charge maximal est compris entre 2 et 15 N, de préférence entre 2,5 et 12 N, de manière encore plus préférée entre 3 et 11 N.

7. Dispositif de fixation et de support selon l'une ou plusieurs des revendications précédentes, **caractérisé par le fait que** ladite portion bavette (32, 34) se rétrécit en direction (38) de la zone d'entrejambe.

8. Dispositif de fixation et de support selon l'une ou plusieurs des revendications précédentes, **caractérisé par le fait que** les éléments de fermeture (40, 42) de la portion bavette sont prévus au moins sur une extrémité de la portion bavette, qui montre vers l'entrejambe.

9. Dispositif de fixation ou de support selon l'une ou plusieurs des revendications précédentes, **caractérisé par le fait que** les éléments de fermeture (40, 42) de la portion bavette (32, 34) sont prévus sur une portion de matière en forme de bande et s'étendant transversalement à la direction longitudinale qui est rapportée à la portion bavette (32, 34).

10. Dispositif de fixation et de support selon l'une ou plusieurs des revendications précédentes, **caractérisé par le fait que** les éléments de fermeture de la portion bavette (32, 34) et/ou les éléments de fermeture de ceinture (22) et/ou les éléments de fermeture secondaires (26) sont des éléments de fermeture à effet mécanique et/ou collant.

11. Dispositif de fixation et de support selon l'une ou plusieurs des revendications précédentes, **caractérisé par le fait que** la ceinture de hanches (6) ne peut être fermée et ouverte que dans une zone et que les éléments de fermeture de ceinture (22, 24, 25) sont prévus dans cette zone sur des portions terminales libres (10, 12) de la ceinture de hanches (6).

12. Dispositif de fixation et de support selon l'une ou plusieurs des revendications précédentes, **caractérisé par le fait que** des éléments de fermeture secondaires (26) sont prévus par le bais desquels on peut régler une longueur circonférentielle de ladite ceinture de hanches et, ainsi, positionner au moins la portion bavette (32) avant de manière symétrique par rapport à l'entrejambe de l'utilisateur.

13. Dispositif de fixation ou de support selon l'une ou plusieurs des revendications précédentes, **caractérisé par le fait que** les éléments de fermeture de ceinture (22, 24, 25) et les éléments de fermeture secondaires (26) sont prévus de part et d'autre de la zone ventrale (14) ou dans des zones latérales (18, 20) de la ceinture de hanches (6).

14. Dispositif de fixation ou de support selon l'une ou plusieurs des revendications précédentes, **caractérisé par le fait que** la zone ventrale (14) et/ou la zone dorsale (16) de la ceinture de hanches (6) est constituée par un matériau de non-tissé ou par un matériau composite de non-tissé.
